Europäisches Patentamt

European Patent Office

Office européen des brevets

(11) Publication number: **0 370 467**
**A2**

# EUROPEAN PATENT APPLICATION

(21) Application number: 89121525.3

(51) Int. Cl.5: **A61K 31/13**

(22) Date of filing: **21.11.89**

(30) Priority: **22.11.88 CS 7641/88**

(43) Date of publication of application:
**30.05.90 Bulletin 90/22**

(84) Designated Contracting States:
**CH DE FR GB LI**

(71) Applicant: **CESKOSLOVENSKA AKADEMIE VED**
**Narodni 3**
**Praha 1(CS)**

(72) Inventor: **Zenka, Jan, RNDr. CSc.**
**Bezdrevská 15**
**C. Budejovice(CS)**
Inventor: **Hulinska, Dagmar, RNDr. CSc.**
**Fetrovská 26**
**Praha 6(CS)**
Inventor: **Holusa, Radim, MUDr. CSc.**
**Kotorská 10**
**Praha 4(CS)**
Inventor: **Pokorny, Theodor, MVDr. CSc.**
**Netolická 11**
**C. Budejovice(CS)**
Inventor: **Jegorov, Alexandr, RNDr. CSc.**
**Bezdrevská 15**
**C. Budejovice(CS)**

(74) Representative: **Patentanwälte Beetz sen. - Beetz jun. Timpe - Siegfried - Schmitt-Fumian- Mayr**
**Steinsdorfstrasse 10**
**D-8000 München 22(DE)**

(54) **Pharmaceutical preparations for improvement of regeneration of injured striated muscles.**

(57) The invention relates to a preparation for improving the course of regeneration of injured striated muscles which comprises n-butylamine in the natural or salt form, or in a buffered aqueous solution, as active substance.

# Pharmaceutical preparations for improvement of regeneration of injured striated muscles

The present invention relates to a therapeutic preparation for the improvement of the regenerative process of injured striated muscles. Under the influence of this preparation the reparative processes and formation of collagen are suppressed, and an injured site regenerates by formation of new muscle fibres.

Until now, no pharmaceutical which would enable regeneration of injured striated muscles (incision, stab and lacerate wounds) has been known. Injuries of striated muscles heal by cicatrization (with the exception of particular injuries of muscles such as compression or application of some myotoxic local anesthetics, e.g. Marcain). The scar is formed mostly by collagen and has no capacity of active contractions, and from the viewpoint of muscle activity it represents only a closure of an injured site. Moreover, it is not aesthetic because it is formed by a mass having different optical properties and remains visible lifelong. In above-mentioned special cases of muscle injuries inside a muscle, without the total muscle being injured, the activation of proliferation of satellite cells and the formation of new muscles take place here. These natural regenerative processes are activated if the muscle is injured mechanically to a great extent (incision, stab and lacerate wounds etc.) but within a fortnight they are fully substituted by a granular tissue which forms the scar.

It is the object of the present invention to provide a pharmaceutically active compound and corresponding pharmaceutical compositions enabling a regeneration of injured striated muscles which suppresses reparative processes and particularly collagen formation and induces regenerative processes.

The above object is achieved according to the claims. The dependent claims relate to preferred embodiments.

The pharmaceutical preparations according to the present invention are characterized in that they consist of or comprise n-butylamine and/or a pharmacologically acceptable ionic derivative thereof, particularly an acid addition salt with an inorganic or organic acid, preferably the hydrochloride.

Accordingly, the above-mentioned failure problem in regeneration of injured striated muscles (incision, stab and lacerate wounds etc.) is overcome by the preparations according to the invention which are characterized by the presence of n-butylamine in natural or ionic form, particularly salt form, or in a buffered solution as active substance. The compositions advantageously comprise the hydrochloride in aqueous solution, advantageously saline. They are preferably administered by injection into the vicinity of the muscle injury. The active compound suppresses reparative processes and formation of collagen, and, in contrast thereto, the regenerative processes are fully developing, the proliferation of satellite muscle cells leading to the formation of new myotubes. At the site of the injury a new muscle mass is forming (striated muscles), i.e. a mass which possesses the same (also optically) properties. After a certain time, the site of the original muscle injury is no longer visible.

By application of the preparation according to the present invention, healing of the muscle injury is reached actually by formation of a muscle mass and not by collagen formation which is suppressed.

In accordance therewith, the significance of this full regeneration is very large. The preparations may be applied for the treatment of muscle injuries both in veterinary and human practice. Moreover, the treatment of muscle injuries in humans is accompanied by a very important aesthetic factor consisting in the fact that no scars are formed in a muscle.

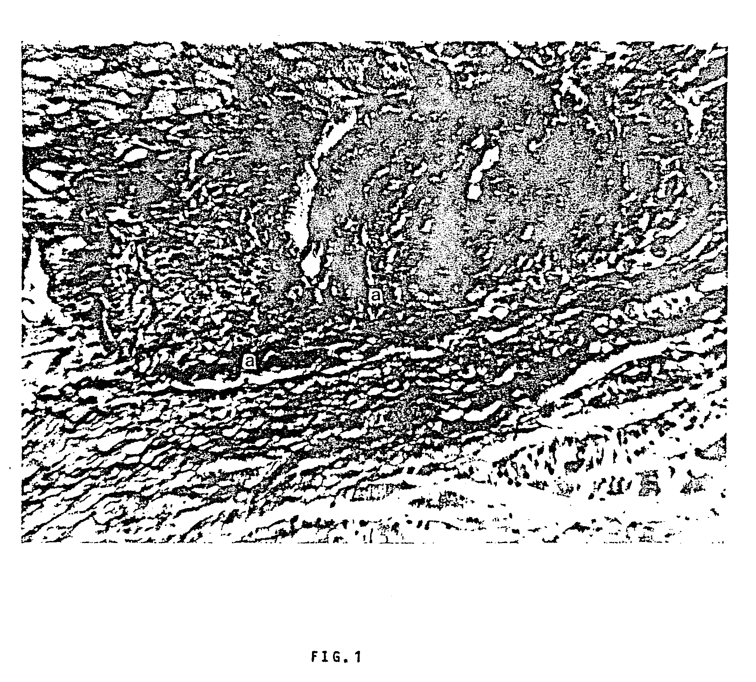

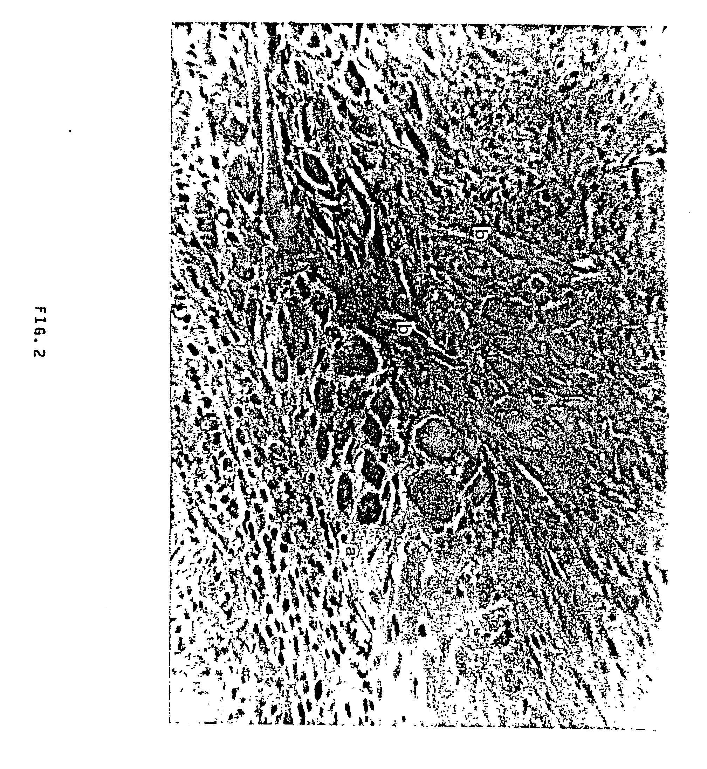

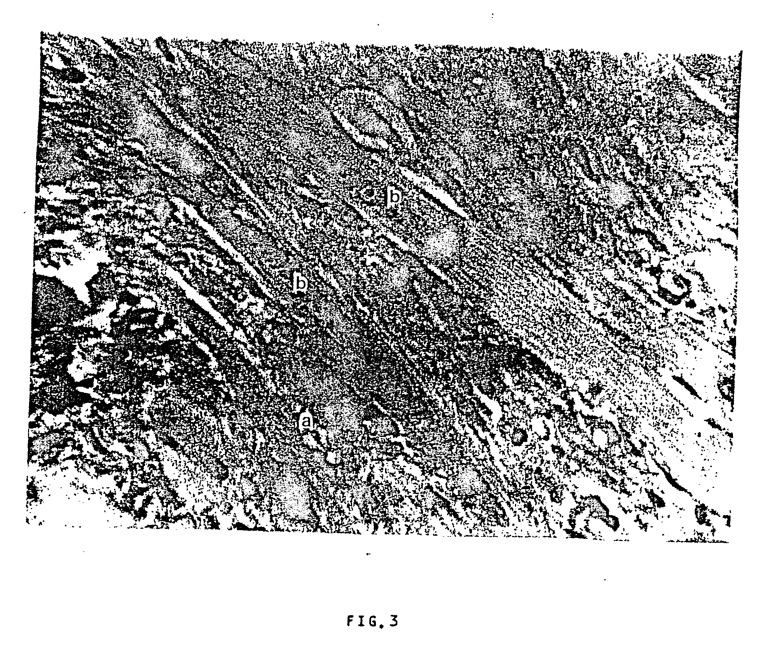

Figures 1 to 3 show microphotographs of results of the examination of an injured muscle obtained with an optical microscope. Both cases, i.e. a muscle healing without any intervention and muscle healing after application of the preparation according to the invention are described below:

Fig. 1 shows a histological section through the scar of an injured muscle without any treatment. A scar formed by collagen (a) represents the normal way of healing (magnification x 160).

Fig. 2 shows a histological section through a injured muscle regenerating under the influence of the preparation according to the invention. Regenerative changes of the muscle can be observed. The proliferation of satellite muscle cells followed by their differentiation leads to the formation of new myotubes (b). Collagen (a) occurs at sites corresponding to uninjured muscle (embedding of endomysium, formation of lamina basalis) (magnification x 160).

Fig. 3 shows a detail of the previous histological preparation of Fig. 2 with well visible new myotubes (b) (magnification x 450).

In the following, the invention will be explained with more details with reference to examples.

Fourteen 2-month-old rats weighing 280-285 g were used in the experiment. A muscle injury was produced on both hindlegs (musculus tibialis anterior). The skin was cut with scissors, the fascia was retracted aside, and the muscle was injured by longitudinal and transverse incisions of 5 mm in length and 5 mm in depth with fine scissors having sharp tips. The skin was then stitched, and the injured site was treated immediately by injecting 0.37 ml of a solution of n-butylamine hydrochloride (BU) in saline (experiment) or of the same volume of saline (controls). This volume was applied with 6 injections into the vicinity of and around the injured site.

The experimental arrangement was as follows:

| Rat no. | Applied | |
|---|---|---|
| | left leg | right leg |
| 1<br>2<br>3<br>4 | 0.37<br>ml<br>of<br>saline | 0.37<br>ml<br>of<br>saline |
| 5<br>6 | 0.37 ml of solution of 38 mg BU /ml of saline | 0.37 ml of saline |
| 7<br>8<br>9<br>10 | 0.37 ml of solution of 76 mg BU/ml of saline | 0.37<br>ml<br>of<br>saline |
| 11<br>12 | 0.37 ml of solution of 152 mg BU/ml of saline | 0.37 ml of saline |
| 13<br>14 | 0.37 ml of solution of 76 mg BU/ml of saline after injury + on 5th and 9th day | 0.37 ml of saline after injury + on 5th and 9th day |
| Rats no. 13 and 14 were also treated with the two solutions on the 5th and 9th day after injury. | | |

The rats were then killed, and assessment of the healing process was made both visually and by means of optical and electron microscopy. The injuries which were treated by injecting around, a solution of n-butylamine hydrochloride were healed with formation of a new muscle mass; sometimes it was difficult to find the very site of injury. A total of 20 samples obtained from each investigated muscle were taken for microscopic examination along the whole length of injury. It was confirmed that in each case where a solution of n-butylamine hydrochloride was applied in the vicinity of the injury, regeneration of striated muscles, proliferation of satellite muscle cells and formation of new myotubes took place there. The formation of collagen was suppressed; only 5 to 20 % of collagen was observed at the site of injury, the remaining mass was formed especially by new myotubes. The injuries of the right hind legs muscles of rats in which n-butylamine hydrochloride penetrated only to a small extent through the blood, and the injured muscles of both hind legs of control rats no. 1 to 4 (treatment in all cases only by injection of saline) healed in a classical manner forming a collagen scar. In these cases, collagen represented 90 to 100 % of the whole mass. New myotubes were found only sporadically, and only a collagen scar was found in the middle of the injury.

It follows from the experiment that concentrative effects and an influence of repeated applications were observed. The effect of the preparation shows a certain concentration dependency; there is a region of optimum effectivity, which can be easily determined, the effect of the preparation being rather decreased at lower or higher concentrations. Application of the solution of a concentration of 76 mg of n-butylamine hydrochloride/ml of saline can be considered as optimum in the experiment. Repeated application (rats no. 13 and 14) improves the result of regeneration.

The invention offers the possibility of application both in veterinary and human practice during treatment of muscle injuries. Another possible application is the suppression of reparative processes which results in a favourable course of regeneration of other tissues than striated muscles, and the use in cosmetic surgery.

If necessary, the active substance according to the present invention may be combined with usual pharmacologically acceptable additives, carriers etc., and also with additional active compounds.

**Claims**

1. n-Butylamine and its pharmaceutically acceptable salts with inorganic and organic acids for use for the treatment of muscle injuries and for the regeneration of other tissues in human and veterinary medicine.

2. Pharmaceutical preparations, characterized in that they consist of or comprise n-butylamine and/or a pharmacologically acceptable ionic derivative thereof, particularly an acid addition salt with an inorganic or organic acid, as active substance.

3. Pharmaceutical preparations according to claim 2, characterized in that the active substance is n-butylamine hydrochloride.

4. Pharmaceutical preparations according to claim 2 and 3, characterized in that the active substance is present in an aqueous solution.

5. Pharmaceutical preparations according to claims 2 to 4, characterized in that the active substance is present in a saline solution.

6. Pharmaceutical preparations according to claims 2 to 5, characterized in that the active substance is present in a buffer solution.

7. Pharmaceutical preparations according to claims 2 to 6, characterized in that they comprise pharmaceutically acceptable additives and/or carriers.

8. Pharmaceutical preparations according to claims 2 to 7, characterized in that they are present in the form of injectable solutions.

9. Use of n-butylamine and of its pharmaceutically acceptable ionic derivatives, particularly the addition salts with inorganic and organic acids, for the preparation of pharmaceutical formulations.

10. Use of n-butylamine and of its pharmaceutically acceptable ionic derivatives, particularly the addition salts with inorganic and organic acids, for the preparation of pharmaceutical formulations for the treatment of muscle injuries, the regeneration of other tissues, and for cosmetic surgery.

4

FIG. 1

EP 0 370 467 A2

FIG. 2

FIG. 3